(19) Europäisches Patentamt
European Patent Office
Office européen des brevets

(11) Numéro de publication : **0 627 702 A1**

(12) **DEMANDE DE BREVET EUROPEEN**

(21) Numéro de dépôt : **94401185.7**

(51) Int. Cl.$^5$ : **G06F 15/72**

(22) Date de dépôt : **30.05.94**

(30) Priorité : **02.06.93 FR 9306564**

(43) Date de publication de la demande : **07.12.94 Bulletin 94/49**

(84) Etats contractants désignés : **BE DE FR GB**

(71) Demandeur : **COMMISSARIAT A L'ENERGIE ATOMIQUE**
**31-33, rue de la Fédération**
**F-75015 Paris (FR)**

(72) Inventeur : **Grangeat, Pierre**
**136 allée des vignes du Persan**
**F-38330 Saint-Ismier (FR)**
Inventeur : **Guillemaud, Régis**
**15 Bld Maréchal Leclerc**
**F-38000 Grenoble (FR)**

(74) Mandataire : **Ilgart, Jean-Christophe et al**
**c/o Société Brevatome,**
**25, rue de Ponthieu**
**F-75008 Paris (FR)**

(54) **Installation et procédé de reconstruction d'images tridimensionnelles.**

(57) Installation et procédé pour obtenir des images tridimensionnelles d'un objet (4) par un réseau plan (1) de détecteurs (2) dont les points de focalisation sont situés sur un cercle (Ce). L'avantage obtenu est un champ de détection plus large qu'avec une focalisation sur un point unique. Des règles sont données pour utiliser les algorithmes d'inversion des mesures caractéristiques de la focalisation conique (sur un point unique).

Application à la médecine et aux contrôles non destructifs de l'industrie.

FIG. 1

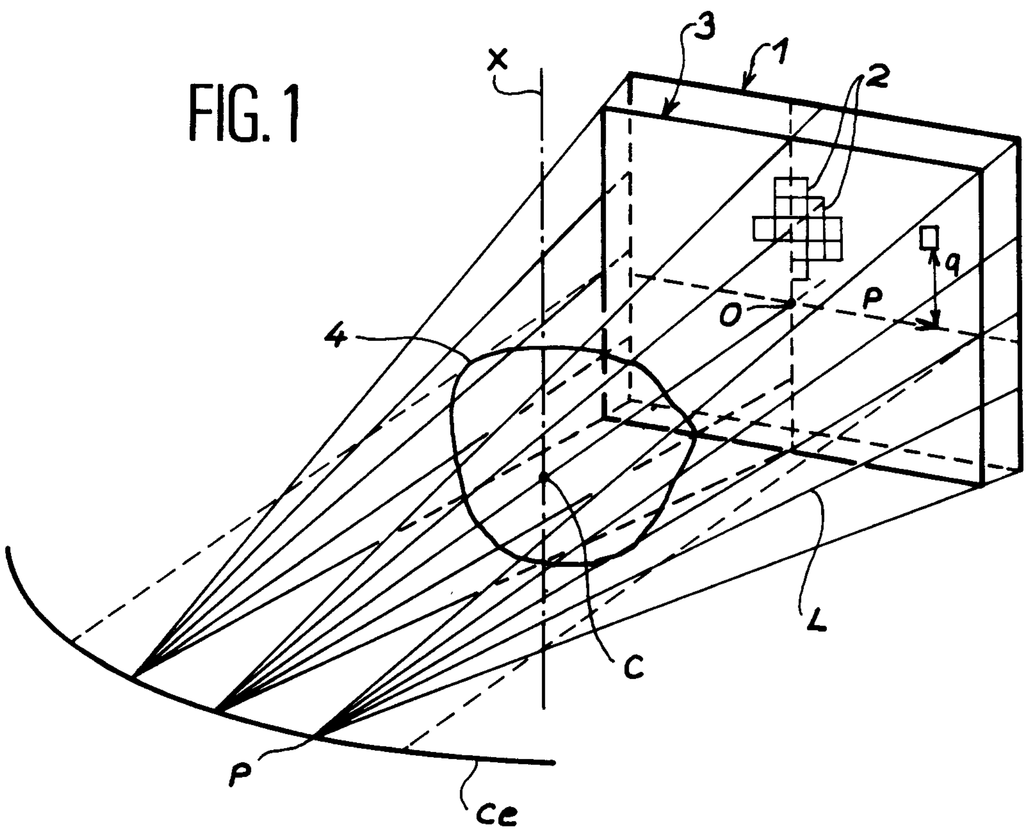


EP 0 627 702 A1

L'invention concerne une installation et un procédé de reconstruction d'images tridimensionnelles.

Les objets dont on doit reconstruire l'image par un procédé de tomographie, notamment en médecine ou dans les contrôles non destructifs de l'industrie, sont placés devant un réseau bidimensionnel de capteurs qui tourne autour d'eux. Le réseau est recouvert d'un collimateur, c'est-à-dire d'une plaque de plomb ou d'un matériau analogue qui limite l'espace scruté par chaque détecteur à une seule ligne. Le réseau est promené sur un cercle ou une autre trajectoire fermée autour de l'objet ou une trajectoire de type hélicoïdale, et la collation des mesures des détecteurs, rassemblées en plusieurs images bidimensionnelles prises selon autant d'incidences différentes, permet de reconstruire une image tridimensionnelle de l'objet par une méthode analytique d'inversion d'une transformée mathématique des mesures ou une résolution algébrique d'inversion d'un système d'équations. La fonction de l'objet ou la propriété qui caractérise l'image et qui est mesurée par les détecteurs est soit l'émission d'un rayonnement par les points de l'objet, soit l'atténuation par les points de l'objet d'un rayonnement émis par une source au-delà de l'objet sur les lignes de focalisation.

Le lieu des points de focalisation des détecteurs, c'est-à-dire des points d'intersection des lignes de focalisation, peut prendre des formes diverses. On a ainsi proposé une collimation purement conique, où le point de focalisation est unique pour chaque image bidimensionnelle, ce qui permet de n'utiliser qu'une source ponctuelle. Les travaux antérieurs menés par l'un des inventeurs et par d'autres chercheurs ont prouvé la valeur de cette solution si on utilise l'inversion de ce qu'on appelle la dérivée première de la transformée de Radon de la fonction pour reconstituer les images (notamment dans le brevet européen 0 292 402) ou plus généralement, les sommes de la fonction sur des plans.

On a encore proposé de focaliser les détecteurs sur une droite parallèle à l'axe de rotation du réseau, c'est-à-dire que les lignes de focalisation sont disposées en éventails sur des plans étagés et parallèles à la trajectoire. Ce mode de focalisation, "éventail parallèle", décrit dans un certain nombre d'articles, est désavantageux à cause de l'absence de grossissement dans l'axe de rotation.

Des lieux de focalisation plus complexes sont proposés dans les brevets américains 4 820 924 et 4 823 017. Les lignes de collimation des rangées horizontales et verticales (lignes et colonnes) de détecteurs convergent pour au moins les unes d'entre elles sur des lignes normales au réseau. Dans une réalisation, le lieu de focalisation est une droite parallèle au réseau et perpendiculaire à l'axe de rotation. Mais les calculs de reconstruction deviennent alors très compliqués.

Enfin, l'article de Müller, Arce et Blake intitulé "Truncation artifacts and synthetic scanner arrays in two-dimensional computerized tomography" et paru dans les comptes-rendus de l'ECAPT à Karlsruhe en 1993 décrit une méthode de reconstruction d'images tridimensionnelles qui comprend une étape de conversion des projections en projections virtuelles afin de réduire les effets de la troncature des mesures. Le réseau de détecteurs permet d'acquérir un ensemble de projections tronquées en géométrie conique, grâce en particulier au mouvement circulaire du réseau de détecteurs, mouvement indépendant de celui de la source, et les projections virtuelles sont effectuées sur un réseau virtuel, plan, de capteurs. On verra que l'invention implique aussi une conversion des projections réelles en projections virtuelles, mais dans des conditions d'acquisition différentes puisque la focalisation est conique ou en éventails parallèles dans cet article.

L'invention concerne une installation dont le lieu de focalisation n'est pas ponctuel mais qui permet cependant d'utiliser avec fort peu d'adaptations les algorithmes de reconstruction d'images associés à des focalisations purement coniques, contrairement aux focalisations en éventails parallèles qui nécessitent des traitements complètement différents.

Un défaut inhérent à la focalisation ponctuelle est en effet que le champ d'examen se rétrécit très vite vers le foyer. Si ce point est relativement proche de l'objet, une partie de l'objet n'est généralement pas incluse dans le champ de rayonnement de toutes les mesures, et on obtient alors des mesures tronquées qui se traduisent par des erreurs de reconstruction qu'il est impossible de corriger dans les formules d'inversion. Si le point focal est écarté de l'objet ou si l'objet est rapproché du réseau de détecteur, il est plus souvent entièrement inclus dans le cône d'examen, mais le grossissement de l'image est plus faible, ce qui est regrettable si on ne s'intéresse qu'à l'examen d'une partie de l'objet tel qu'un organe du corps humain, car la plupart des détecteurs sont consacrés à des mesures qui concernent les autres parties de l'objet.

L'invention concerne, pour résumer, une installation de reconstruction d'images tridimensionnelles d'un objet, comprenant un réseau bidimensionnel de détecteurs orientés vers l'objet, le réseau de détecteurs étant mobile autour de l'objet en tournant autour d'un axe suivant une trajectoire hélicoïdale ; elle est caractérisée en ce que les détecteurs sont focalisés, suivant une direction axiale, sur une pluralité de points sensiblement situés sur une hélice centrée sur l'axe, et des moyens connus en eux-mêmes de mémorisation de mesures prises par les détecteurs et de combinaison des mesures sont utilisés pour reconstruire les images. Par combinaison des mesures, en entend une série d'étapes telles que par exemple des réarrangements et des traitements des mesures et les procédés de reconstruction d'images tridimensionnelles. Le prisme d'examen est alors beaucoup plus large qu'un cône. Il est possible que la trajectoire soit circulaire et l'hélice un cercle, ce

qui correspond à un pas nul d'hélice. Ce cas a une grande importance pratique car les appareils sont généralement conçus pour accomplir de telles trajectoires mais il ne diffère presque pas en réalité du cas général pour le procédé de reconstruction et les calculs.

Les détecteurs du réseau situés sur une colonne parallèle à l'axe sont de préférence tous focalisés sur un point unique parmi les points de focalisation, afin de faciliter les calculs.

L'invention concerne aussi le procédé qui offre la possibilité de se servir des mesures prises notamment avec l'installation définie ci-dessus en les convertissant en données caractéristiques d'une focalisation purement conique qui sera appelée une focalisation virtuelle. Cette conversion ne porte pas sur les valeurs des mesures elles-mêmes, mais sur les paramètres géométriques qui les définissent. Sous sa forme la plus générale, l'invention concerne alors un procédé de reconstruction d'images tridimensionnelles d'un objet consistant à prendre des images bidimensionnelles de l'objet par au moins un réseau bidimensionnel de détecteurs mobile autour de l'objet en tournant autour d'un axe en suivant une trajectoire hélicoïdale, les images bidimensionnelles étant composées de points de mesure pour chacun des détecteurs d'une propriété de l'objet le long de lignes de collimation, les images tridimensionnelles étant reconstruites par une méthode de résolution d'équations utilisant les mesures, caractérisé en ce que chacune des lignes de collimation passe sensiblement sur une hélice centrée sur l'axe et en ce que le procédé de reconstruction comporte avant la méthode de résolution une étape de calculs de conversion des paramétrages des lignes de collimation, où chacune des lignes de collimation est supposée être originaire d'un réseau virtuel bidimensionnel de détecteurs focalisé vers un point focal unique de l'hélice, le réseau virtuel tournant autour de l'axe et à distance constante de l'axe, les calculs de conversion consistant à déterminer pour chacune des lignes de collimation du réseau virtuel, l'endroit du réseau réel dont elle est originaire et la position angulaire du réseau réel autour de l'axe ; il existe ensuite une étape de regroupement de mesures selon les déterminations des calculs de conversion, de façon à obtenir des projections coniques virtuelles, la résolution concernant les mesures regroupées ; la méthode de résolution des mesures comporte avantageusement une inversion de la dérivée première de la transformée de Radon de la fonction qui caractérise l'image de l'objet.

Il est préférable, pour la netteté de l'image obtenue, que toutes les lignes de focalisation passent par l'hélice ou éventuellement le cercle, mais cette condition n'est pas obligatoire car la dégradation de l'image n'est que peu sensible si ces lignes passent un peu à l'écart du cercle. Des résultats satisfaisants ont été constatés si toutes les lignes de focalisation passaient par une droite tangente à un cercle, conformément à des collimateurs déjà connus.

Le pas de l'hélice de focalisation peut être identique à celui de la trajectoire, sans que cela soit nécessaire.

L'invention va maintenant être décrite à l'aide des figures suivantes qui en détaillent une réalisation à titre illustratif et non limitatif :

- la figure 1 représente une vue générale et schématique de l'invention,
- la figure 2 donne la répartition des lignes de focalisation dans une réalisation préférée en vue suivant l'axe,
- les figures 3 et 4 explicitent la méthode utilisée pour se ramener à la reconstruction d'images d'après une focalisation conique,
- la figure 5 explique comment l'invention peut être mise en oeuvre dans des conditions un peu différentes de la focalisation sur un cercle,
- et la figure 6 décrit une généralisation de la méthode.

On a représenté sur la figure 1 le réseau 1 de détecteurs 2, qui sont alignés sur des lignes perpendiculaires à l'axe de rotation et des colonnes parallèles à l'axe de rotation et qu'on peut identifier par leurs coordonnées de lignes et colonnes p et q dans le plan du réseau 1, comptées à partir du centre O du réseau 1.

Un collimateur 3 recouvre le réseau 1. Il n'est pas illustré en détail mais comprend de manière classique une quantité de fenêtres percées dans une matière opaque au rayonnement que les détecteurs 2 doivent mesurer. Du plomb est généralement employé. Les fenêtres sont en nombre supérieur ou égal à celui des détecteurs 2, et placées devant chacun d'eux pour les rendre aptes à scruter l'espace sur une ligne de focalisation L orientée vers l'objet 4 à examiner et au-delà.

Avantageusement, le collimateur comprend un nombre de fenêtres supérieur au nombre de détecteurs.

Avantageusement encore, les fenêtres sont de même taille et réparties de façon homogène sur la surface du collimateur.

Les fenêtres placées devant un détecteur ont des directions de perçage voisines, de façon à rendre les détecteurs aptes à scruter l'espace sur une ligne de focalisation L unique. La direction de la ligne L de focalisation est une direction moyenne des directions des fenêtres se trouvant devant le détecteur.

On appelle X l'axe de rotation du réseau 1. La caractéristique essentielle de l'invention consiste en ce que les lignes de focalisation L aboutissent toutes à un cercle Ce situé autour de l'objet 4 et qui appartient à un plan perpendiculaire à l'axe X, qui passe avantageusement d'ailleurs par le centre C du cercle Ce. Dans le

cas d'une trajectoire de type hélicoïdal, les lignes de focalisation L aboutissent toutes à un arc d'hélice. Ce plan est appelé plan de focalisation. De plus, les lignes de focalisation L de tous les détecteurs 2 situés sur chaque colonne convergent vers un point unique P respectif. On voit immédiatement que cette disposition des points de focalisation P élargit sensiblement le champ de l'espace scruté et que l'objet 4 y est beaucoup plus facilement inclus, si bien que la contribution de ses points à l'atténuation ou à l'émission du rayonnement est plus équitablement mesurée. Un collimateur permettant une telle disposition des lignes de focalisation est appelé collimateur à géométrie multifocale ou collimateur multifocal. Un tel collimateur peut remplacer des collimateurs de type parallèle ou conique utilisés sur les tomographes actuels.

La figure 2 représente une projection orthogonale du réseau 1 et des lignes de focalisation L sur le plan de focalisation . Si on appelle F l'axe focal, c'est-à-dire l'axe diamétral du cercle Ce qui passe par l'axe X et par le centre O du réseau 1, f la distance mesurée sur l'axe focal F entre le cercle Ce et le réseau 1, les projections des lignes de focalisation Lm dans le plan de focalisation coupent l'axe focal F en une pluralité de joints focaux transversaux Ftm. Si on considère une ligne de focalisation Lm, il est avantageux que, si la coordonnée-ligne dudit détecteur 2m est pm, la distance focale transversale D(pm) entre le point focal transversal Ftm et le réseau 1 soit définie par une équation quadratique selon pm ou du moins croissante et supérieure à f, soit d'après l'équation (1)

$$D(pm) = f + k.pm^2 \quad (1),$$

où k est un coefficient constant.

En effet, on obtient alors un bon compromis entre la nécessité d'embrasser toute la largeur de l'objet 4, ce qui est fait grâce aux lignes de focalisation L extérieures qui sont peu convergentes, et l'intérêt fréquent d'obtenir une meilleure résolution et une meilleure sensibilité pour le centre de l'objet 4, ce qui est réalisé grâce aux lignes de focalisation L centrales qui convergent beaucoup plus.

Si on revient maintenant à la figure 1, on peut induire qu'une série de mesures prises par le réseau 1 autour de l'axe X et de l'objet 4 fournira un nombre important de mesures par les détecteurs 2 et qu'il sera possible de les arranger pour reconstruire l'image tridimensionnelle de l'objet 4 selon une méthode analogue à celle qui existe déjà pour les focalisations coniques.

Voici maintenant comment on y parvient effectivement en ramenant toutes les mesures à des mesures effectuées avec une focalisation virtuelle purement conique. On a représenté sur la figure 3, pour une position du réseau 1, la ligne de focalisation Lm d'un détecteur 2m orientée vers un point de focalisation P. La distance du réseau 1 au cercle Ce, dont le rayon est R, est comme précédemment désignée par f. Si on imagine un réseau de détecteurs virtuel 101 à focalisation conique, tous ses détecteurs convergent vers le point P, et si on le choisit de plus à une distance f du point P et la ligne passant par P et par C étant perpendiculaire au réseau virtuel 101, ce réseau virtuel 101 est alors unique ; la ligne de focalisation Lm se prolonge jusqu'à lui, à une distance pc suivant l'axe des lignes et qc suivant l'axe des colonnes de son centre O', défini comme la projection orthogonale du point P sur le réseau virtuel 101. L'angle que font les deux réseaux 1 et 101 est $\alpha(pm)$.

Il suffit de trouver les relations entre pc et pm, qc et qm, qm étant les coordonnées-colonne du détecteur 2m suivant l'autre coordonnée des réseaux 1 et 101 (figure 4), ainsi que l'angle $\alpha(pm)$ pour convertir les mesures effectuées par le réseau 1 en mesures effectuées par le réseau virtuel 101 et appliquer un algorithme d'inversion connu tel que ceux qui utilisent la dérivée première de la transformée de Radon. La constatation de l'existence de triangles semblables conduit à l'équation

$$t = pc \frac{R}{\sqrt{f^2 + pc^2}} \quad (2)$$

où t est la distance entre le centre C et la ligne Lm et si on appelle $\psi m$ et $\psi c$ les orientations des réseaux 1 et 101 par rapport à un axe de référence fixe du plan de focalisation, l'équation :

$$\Theta = \Psi c - \operatorname{arctg}\left(\frac{f}{pc}\right) \qquad (3)$$

où $\Theta$ désigne l'angle entre l'axe de référence et la projection du centre du cercle Ce sur la ligne de focalisation Lm, peut être définie. Si on introduit la distance D(pm), les équations (2) et (3) sont équivalentes aux équations (4) et (5) :

$$t = pm \frac{D(pm) - (f - R)}{\sqrt{D(pm)^2 + pm^2}} \quad (4)$$

$$\Theta = \Psi m - \text{arctg}\left(\frac{D(pm)}{pm}\right) \qquad (5).$$

A partir des équations (2) et (4), on obtient la relation entre pm et pc

$$pm\,\frac{D(pm) - (f - R)}{\sqrt{D(pm)^2 + pm^2}} = pc\,\frac{R}{\sqrt{f^2 + pc^2}}.$$

On calcule alors pm en fonction de pc.

On peut définir une table de correspondance qui peut être préétablie.

A partir des équations (3) et (5), on obtient :

$$\Psi m = \Psi c - \text{arctg}\left(\frac{f}{pc}\right) + \text{arctg}\left(\frac{D(pm)}{pm}\right) \text{ soit}$$

$$\text{arctg}\left(\frac{D(pm)}{pm}\right) - \text{arctg}\left(\frac{f}{pc}\right) = \delta\Psi(pc).$$

On peut établir une table de correspondance appelée $\delta\Psi$.

On définit le changement de variable entre $\Psi$m et $\Psi$c en utilisant la fonction $\delta\Psi$(pc) de la façon suivante :

$$\Psi m = \Psi c + \delta\Psi(pc)$$

La figure 4 montre immédiatement que qc/qm=fc/fm (6), où fc et fm désignent les longueurs des projections de la ligne de focalisation Lm sur le plan de focalisation, entre le point P et, respectivement, le réseau 1 réel et le réseau virtuel 101. Or fm et fc sont donnés par les équations (7) et (8) :

$$fm = (pm - R\sin\alpha(pm))\sqrt{1 + \left(\frac{D(pm)}{pm}\right)^2} \qquad (7)$$

$$fc = \sqrt{f^2 + pc^2} \quad (8):$$

Une table de correspondance $\delta$q est établie. Le changement de variable entre qm et qc est établi en utilisant la fonction $\delta$q(pc) telle que qm = qc x $\delta$q(pc) et $\delta$q(pc) = fc / fm : qm est donc obtenu en fonction de qc. En pratique, pour chaque point du volume de mesures virtuelles $\Psi$c, pc, qc, les coordonnées du volume de mesures réelles : $\Psi$m, pm, qm correspondantes issues des équations précédentes, ne sont pas entières, c'est-à-dire qu'elles ne correspondent pas à des points de mesure. La détermination de la valeur du volume de mesures réelles au point $\Psi$c, pc et qc est réalisée par des interpolations trilinéaires ou des séries de trois interpolations linéaires successives à une dimension qui dépendent toutes de D(pm), du pas de l'hélice, de la distance focale f, de la distance entre le détecteur et l'axe de rotation R et, respectivement, de pm, de $\Psi$m et de qm. Les mesures réelles sont alors associées à des mesures virtuelles effectuées par le réseau virtuel 101 se déplaçant autour de l'objet 4 et caractérisées par $\psi$c, pc et qc ; ces mesures virtuelles sont combinées, transformées et inversées par un procédé tel que celui du brevet EP-A-O 292 402 dans lequel on décrit également succinctement les moyens, essentiellement informatiques, de mémorisation des mesures, de mise en oeuvre de combinaisons pour effectuer l'inversion de la dérivée première de la transformée de Radon de la fonction et d'affichage des résultats.

On utilise en pratique les formules précédentes ou tout autre ensemble de formules qui permet de passer des mesures réelles multifocales aux mesures virtuelles coniques. Il est dans tous les cas nécessaire de connaître D(pm), et il est possible d'établir à l'avance une tabulation de D(pm) en fonction de pm d'après les caractéristiques géométriques immuables de la focalisation illustrées sur les figures 1 et 2. La recherche de pm à partir de pc et D(pm), qui dépend précisément de pm, n'est pas toujours immédiate et c'est pourquoi on pourra préférer établir à l'avance les tables de pm, $\delta\Psi$ et $\delta$q en fonction de pc, plutôt que de refaire les calculs

pour chacune des mesures. En pratique, lesdites tables étant établies pour un objet à reconstruire de dimension $N^3$, l'étape de réarrangement nécessite de l'ordre de $N^3$ interpolations trilinéaires alors que l'étape de reconstruction nécessite de l'ordre de $N^4$ opérations élémentaires décrites dans le brevet EP-A-0 292 402.

Ces tables pm, $\delta\Psi$ et $\delta$q seront utilisées ultérieurement pour le changement de variables pm, qm, $\Psi$m en fonction de pc, qc et $\Psi$c dans l'étape de conversion des mesures réelles multifocales en mesures virtuelles coniques.

L'alourdissement du procédé qui résulte de ces conversions est cependant assez peu sensible et compte généralement moins que la meilleure qualité d'image obtenue dans nombre d'applications.

Un dispositif concret peut comprendre un collimateur recouvrant un réseau de détecteurs rectangulaires de 450 mm de largeur et 350 mm de hauteur (en direction de l'axe X). Il permet de respecter la situation de la figure 2 et de l'équation (1) avec k = 0,016. Comme de plus R = 350 mm et f = 600 mm, D(pm) varie de 600 à 1410 mm. Le réseau de détecteurs appartient à une caméra gamma permettant de faire des acquisitions en rotation autour du patient (par exemple un tel dispositif est réalisé à partir de la partie mécanique de la caméra DSX de Sopha Médical).

Pour faire des acquisitions suivant une trajectoire hélicoïdale, on applique, en plus du mouvement de rotation du réseau de détecteurs, un mouvement de translation du patient suivant l'axe de rotation. Pour simplifier la description de l'invention, les équations sont données dans la suite, pour des mesures acquises par un détecteur plan. Néanmoins, un détecteur circulaire peut être utilisé, et dans ce cas on détermine la répartition des droites d'acquisition sur le plan défini par la droite reliant les deux extrémités du détecteur et la largeur du détecteur.

L'algorithme de reconstruction d'images est avantageusement une inversion de la dérivée première de la transformée de Radon, appelé algorithme de Grangeat, comme on l'a déjà signalé.

Si les points de focalisation P sont avantageusement situés sur un cercle Ce de centre C appartenant à l'axe X de rotation du réseau 1, cette condition n'est cependant pas obligatoire car les écarts se traduisent par une dégradation de la netteté de l'image qui reste modérée et acceptable en général s'ils sont peu importants. C'est ainsi que les points de focalisation peuvent être situés sur un cercle dont le centre n'est pas confondu avec l'axe X de rotation, mais proche de lui, ce qui résulte dans la réalité des incertitudes de position et de montage du réseau 1 dans le reste de l'appareil. Une situation différente est évoquée à la figure 5, similaire à la figure 2 mais où les points de focalisation P sont placés sur une droite Y parallèle au réseau 1 et perpendiculaire à l'axe X. On commet alors une approximation en supposant que les points de focalisation P sont situés sur un cercle, et par exemple sur le cercle Ce. Les points obtenus dans ces conditions sont dits projetés et référencés par P'. Ils correspondent concrètement à l'intersection du cercle Ce et de la projection parallèlement à l'axe X sur le plan du cercle Ce de chacune des lignes de focalisation L. Les points de focalisation P sont presque confondus avec les points projetés P' au centre de l'image, ce qui implique que l'image tridimensionnelle reconstruite sera plus nette pour cette région qui englobe précisément l'objet 4 qu'à la périphérie de l'image.

Le procédé décrit peut être complété par des traitements préliminaires tels que des corrections d'uniformité et de sensibilité des détecteurs 2, des traitements ultérieurs tels que des corrections d'atténuation. Ce procédé peut être mis en oeuvre dans des reconstructions classiques dans ce domaine et dont on trouvera l'illustration notamment dans le livre "Physics in nuclear medicine" de J.A. Sorenson et M.E. Phelps, aux éditions Saunders.

On a en réalité donné les explications nécessaires sur le cas le plus simple jusqu'à présent : l'invention peut être généralisée à des trajectoires hélicoïdales où le réseau de détecteurs s'élève peu à peu autour de l'objet 4, ce que représente la figure 6 où la trajectoire, notée T, est une hélice de pas h entre ses spires. Cette hélice est centrée sur l'axe X. Le lieu des points de focalisation P du collimateur 3 décrit alors une autre hélice de même pas h, qui est notée H et dont on a figuré une portion ; cette hélice est également centrée sur l'axe X. Il est clair que la méthode de reconstruction reste la même car chaque point de focalisation P peut encore être associé à des détecteurs 2 de colonnes différentes ; l'influence du pas h n'intervient qu'à la fin des calculs, pour reconstruire l'image en fonction de l'instant de chaque prise d'image. La trajectoire circulaire et le cercle Ce correspondent simplement à un pas nul d'hélice.

## Revendications

1. Installation de reconstruction d'images tridimensionnelles d'un objet (4), comprenant au moins un réseau (1) bidimensionnel de détecteurs (2) orientés vers l'objet (4), le réseau de détecteurs étant mobile autour de l'objet en tournant autour d'un axe (X) suivant une trajectoire hélicoïdale (T), caractérisée en ce que les détecteurs (2) sont focalisés suivant une direction axiale sur une pluralité de points (P) situés sensiblement sur une hélice (H) centrée sur l'axe (X) et des moyens de mémorisation de mesures prises par les détecteurs et de combinaison des mesures pour reconstruire les images.

2. Installation suivant la revendication 1, caractérisée en ce que les détecteurs du réseau situés sur une ligne parallèle à l'axe sont tous focalisés sur un point unique des points de focalisation.

3. Installation suivant l'une quelconque des revendications 1 ou 2, caractérisée en ce que la trajectoire hélicoïdale est une trajectoire circulaire et l'hélice est un cercle (Ce).

4. Installation suivant la revendication 3, caractérisée en ce que, sur le plan du cercle (Ce) des points de focalisation (P), les distances (D(pm)), sur la normale (F) au réseau de détecteurs passant par le centre du cercle (C), entre le réseau de détecteurs et les intersections des lignes de focalisation (Lm) avec ladite normale (F), sont supérieures au rayon du cercle (Ce).

5. Installation suivant la revendication 4, caractérisée en ce que lesdites distances sont une fonction croissante des distances (pm) mesurées dans ledit plan entre ladite normale et les détecteurs (2m) dont les lignes de focalisation sont respectivement issues.

6. Installation suivant la revendication 5, carctérisée en ce que la fonction croissante est une fonction quadratique.

7. Procédé de reconstruction d'images tridimensionnelles d'un objet (4) consistant à prendre des images bidimensionnelles de l'objet (4) par au moins un réseau (1) bidimensionnel de détecteurs (2) mobile autour de l'objet en tournant autour d'un axe en suivant une trajectoire hélicoïdale, les images bidimensionnelles étant composées de points de mesure par chacun des détecteurs (2) d'une propriété de l'objet (4) le long de lignes de collimation (L), les images tridimensionnelles étant reconstruites par une méthode de résolution de mesures, caractérisé en ce que chacune des lignes de collimation passe sensiblement sur une hélice centrée sur l'axe et en ce que le procédé de reconstruction comporte avant la méthode de résolution une étape de calculs de conversion où chacune des lignes de collimation est supposée être originaire d'un réseau virtuel (101) bidimensionnel de détecteurs focalisé vers un point focal unique de l'hélice, le réseau virtuel tournant autour de l'axe et à distance constante de l'axe (X), les calculs de conversion consistant à déterminer pour chacune des lignes de collimation du réseau virtuel l'endroit du réseau réel dont elle est originaire et la position angulaire du réseau réel autour de l'axe, et en ce qu'il existe ensuite une étape de regroupement des mesures virtuelles selon les déterminations des calculs de conversion, la résolution concernant les mesures regroupées.

8. Procédé de reconstruction d'images tridimensionnelles suivant la revendication 7, caractérisé en ce que la méthode de résolution des mesures comporte une inversion de la dérivée première de la transformée de Radon.

9. Procédé de reconstruction d'images tridimensionnelles suivant l'une quelconque des revendications 7 ou 8, caractérisé en ce que la trajectoire hélicoïdale est une trajectoire circulaire et l'hélice est un cercle (Ce).

10. Procédé de reconstruction d'images tridimensionnelles suivant la revendication 9, caractérisé en ce que toutes les lignes de focalisation passent par une droite tangente au cercle.

11. Procédé de reconstruction d'image tridimensionnelles suivant la revendication 7, caractérisé en ce que les calculs de conversion comprennent trois étapes successives de conversion des mesures virtuelles suivant trois coordonnées (p, q, $\Psi$), chacune desdites étapes comprenant une interpolation linéaire.

FIG. 1

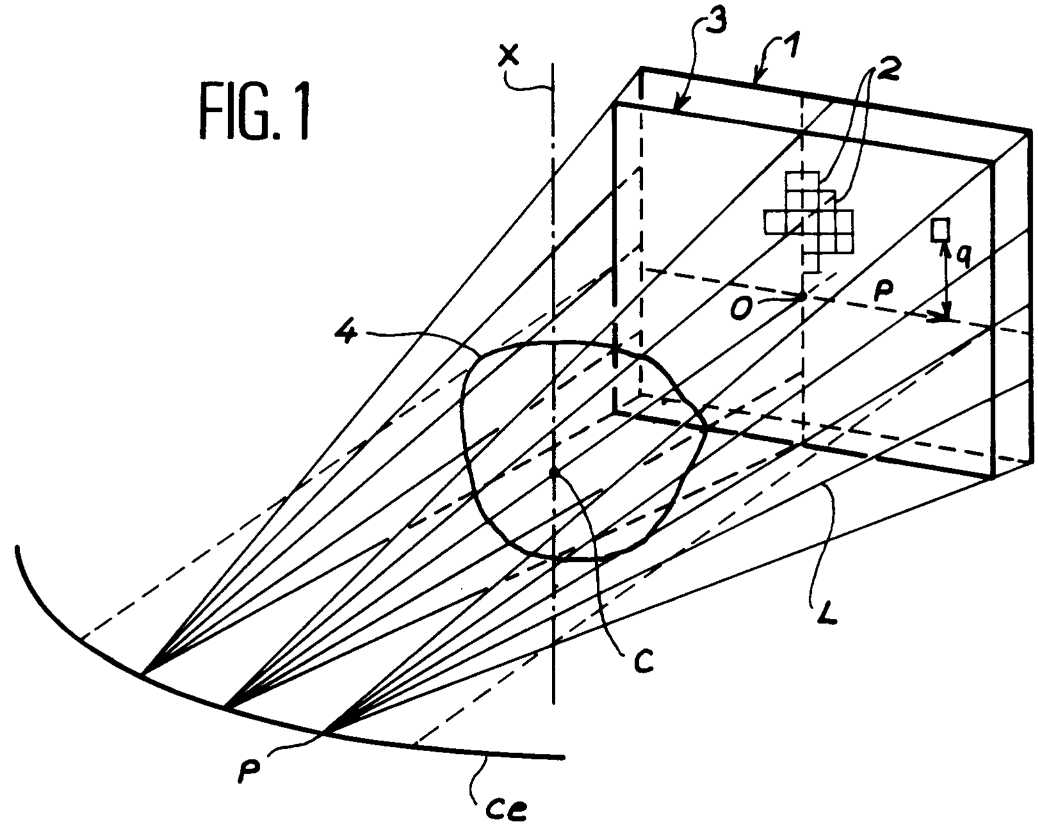


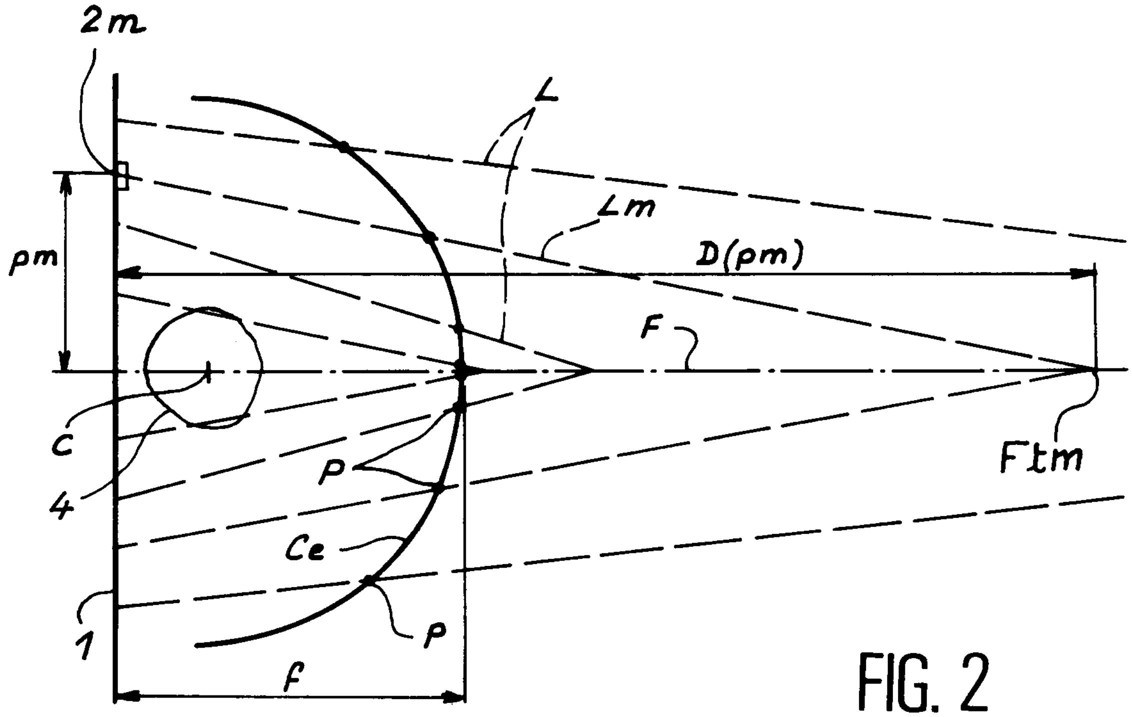


FIG. 2

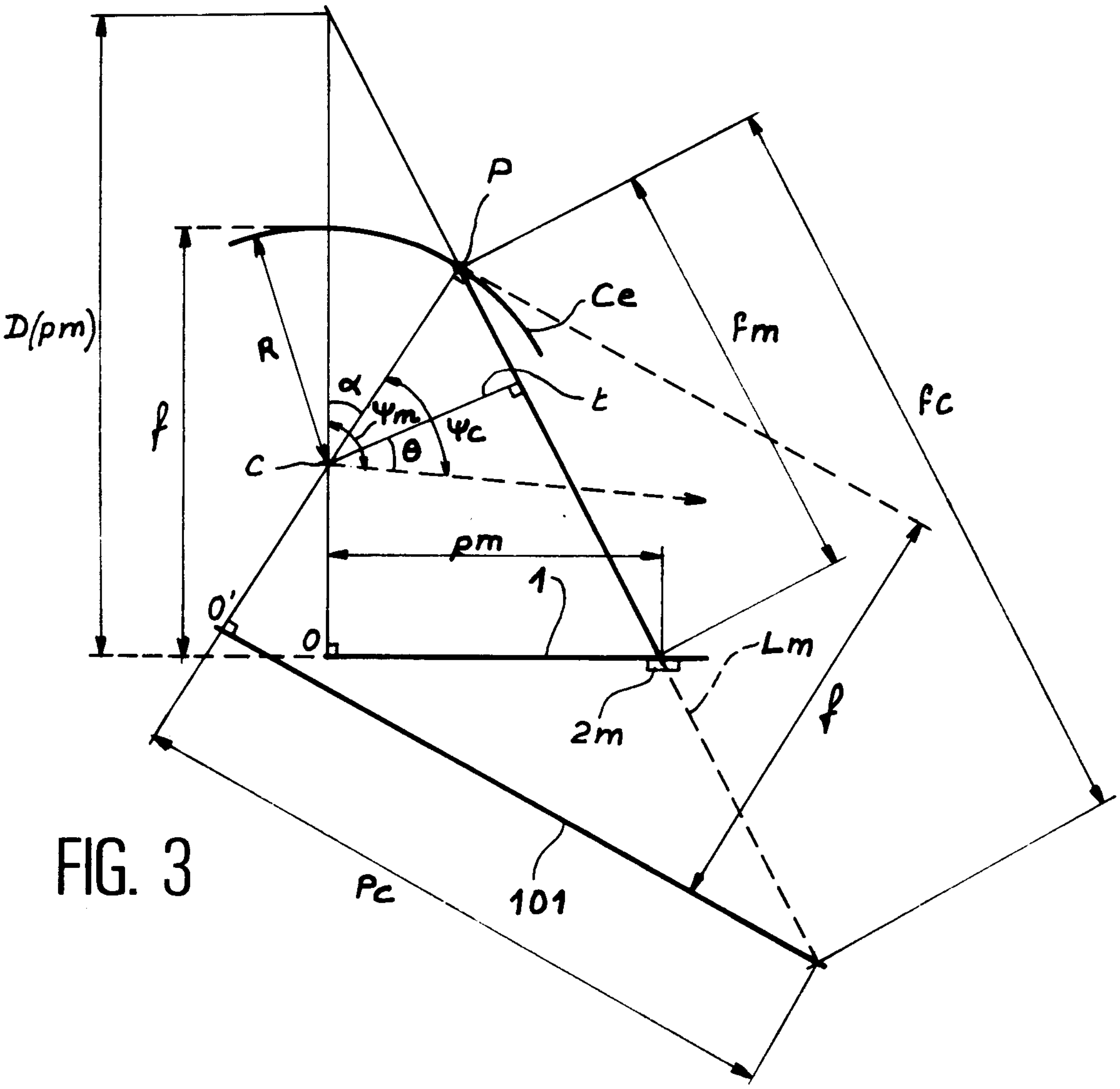
P
Ce
fm
fc
D(pm)
R
α
Ψm
Ψc
t
f
C
θ
pm
1
O'
O
Lm
2m
f
Pc
101

FIG. 3

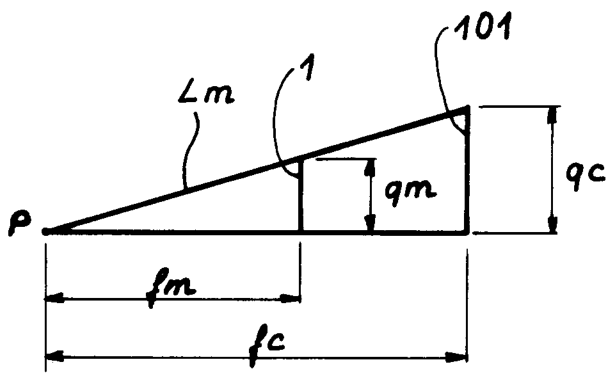
101
1
Lm
P
qm
qc
fm
fc

FIG. 4

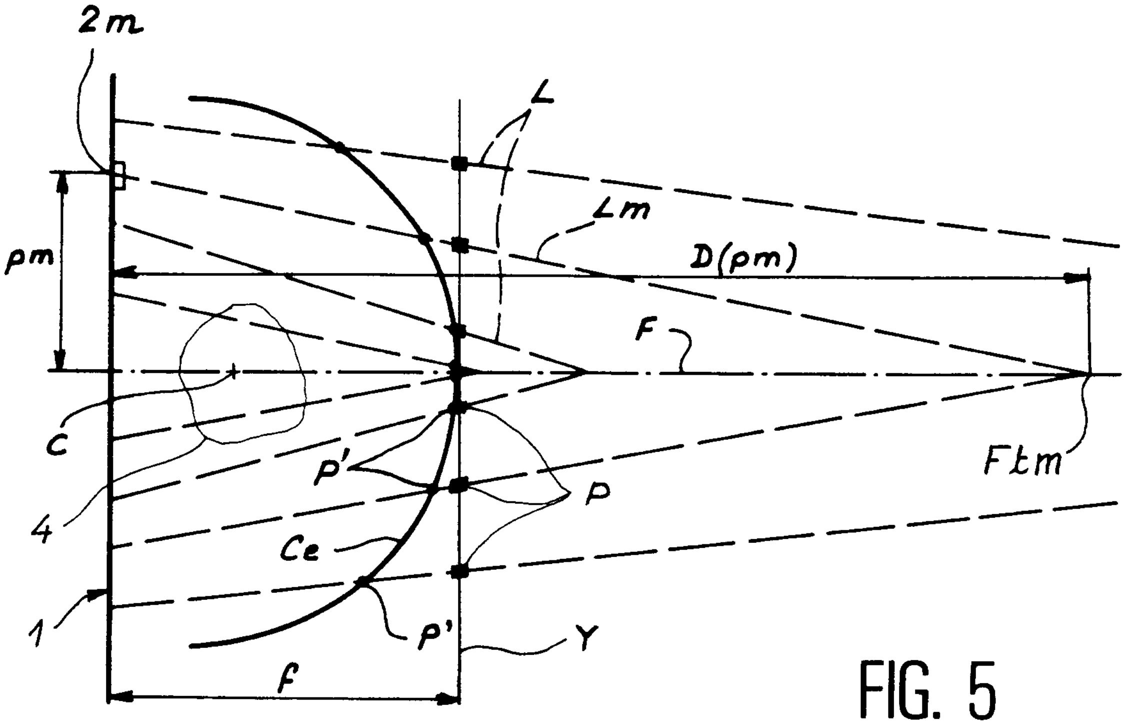


FIG. 5

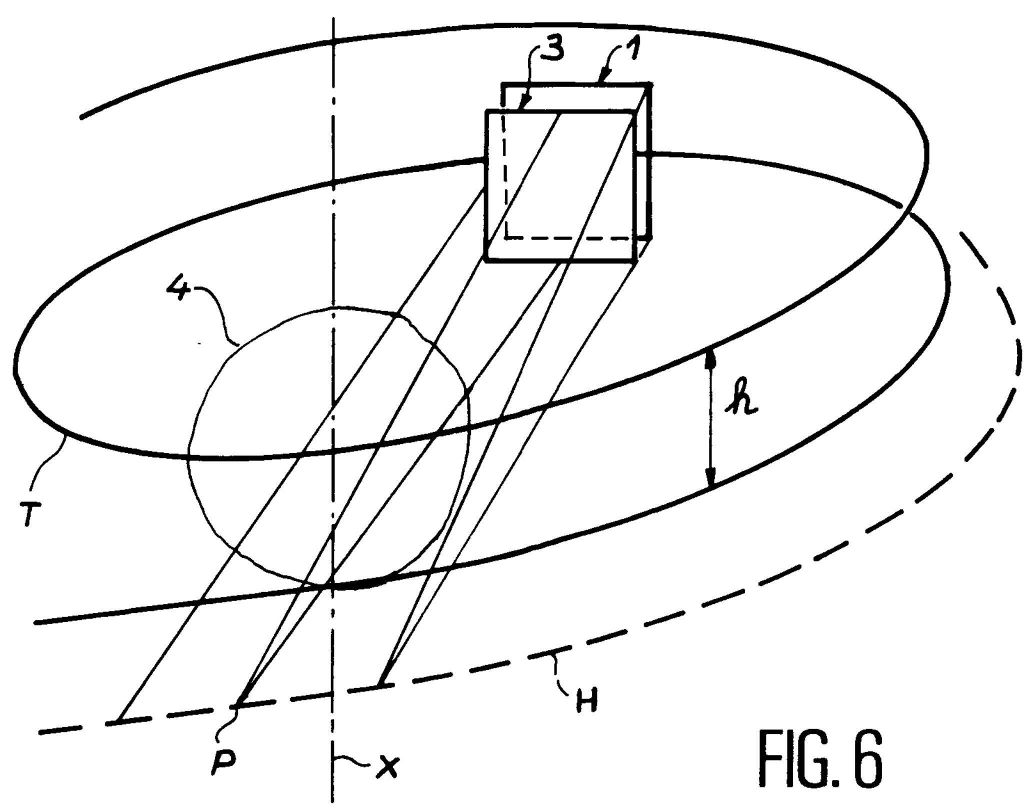


FIG. 6

Office européen des brevets

# RAPPORT DE RECHERCHE EUROPEENNE

Numero de la demande
EP 94 40 1185

| Catégorie | DOCUMENTS CONSIDERES COMME PERTINENTS<br>Citation du document avec indication, en cas de besoin, des parties pertinentes | Revendication concernée | CLASSEMENT DE LA DEMANDE (Int.Cl.5) |
|---|---|---|---|
| Y | SYSTEMS AND COMPUTERS IN JAPAN<br>vol. 23, no. 12 , 1992 , USA<br>pages 75 - 82 XP380347<br>KUDO ET AL. 'three-dimensional helical-scan computed tomography using cone-beam projections'<br>*paragraph: "2.helical scanning using cone-beam projections"*<br>* figure 1 *<br>--- | 1-3 | G06F15/72 |
| Y | EP-A-0 526 970 (THE UNIVERSITY OF UTAH) 10 Février 1993<br>* page 7, ligne 27 - ligne 35; figures 9,10 *<br>--- | 1-3 | |
| A | IEEE TRANSACTIONS ON NUCLEAR SCIENCE<br>vol. 38, no. 2 , Avril 1991 , USA<br>pages 693 - 702 XP229890<br>ZENG ET AL. 'three-dimensional iterative reconstruction algorithms with attenuation and geometric point response correction'<br>--- | | DOMAINES TECHNIQUES RECHERCHES (Int.Cl.5) |
| A | TREZIèME COLLOQUE SUR LE TRAITEMENT DU SIGNAL ET DES IMAGES 16 Septembre 1991 , FR<br>pages 817 - 820 XP242901<br>GRANGEAT ET AL. 'recentes evolutions de la tomographie 3d en geometrie conique'<br>----- | | G06F |

Le présent rapport a été établi pour toutes les revendications

| Lieu de la recherche | Date d'achèvement de la recherche | Examinateur |
|---|---|---|
| LA HAYE | 29 Juin 1994 | Perez Molina, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)